(19) **Europäisches Patentamt European Patent Office Office européen des brevets**

(11) **EP 4 221 692 B1**

(12) **EUROPEAN PATENT SPECIFICATION**

(45) Date of publication and mention of the grant of the patent:
**20.12.2023 Bulletin 2023/51**

(21) Application number: **21783007.4**

(22) Date of filing: **30.09.2021**

(51) International Patent Classification (IPC):
**A61K 9/16** *(2006.01)*      **A61K 9/14** *(2006.01)*
**A61K 31/506** *(2006.01)*

(52) Cooperative Patent Classification (CPC):
**A61K 9/146; A61K 9/1635; A61K 9/1652; A61K 9/1694; A61K 31/506; A61K 31/5377**

(86) International application number:
**PCT/EP2021/076996**

(87) International publication number:
**WO 2022/069661 (07.04.2022 Gazette 2022/14)**

(54) **ACETIC ACID AS PROCESSING AID IN SPRAY DRYING FOR BASIC DRUGS**

ESSIGSÄURE ALS VERARBEITUNGSHILFSMITTEL FÜR DIE SPRÜHTROCKNUNG VON GRUNDARZNEIMITTELN

ACIDE ACÉTIQUE EN TANT QU'AGENT DE TRAITEMENT DE SÉCHAGE PAR PULVÉRISATION POUR MÉDICAMENTS BASIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.10.2020  US 202063086691 P**
**08.10.2020  EP 20200895**
**20.11.2020  EP 20208785**
**11.01.2021  EP 21150829**
**04.06.2021  EP 21177685**

(43) Date of publication of application:
**09.08.2023 Bulletin 2023/32**

(73) Proprietor: **Lonza Bend Inc.**
**Bend, OR 97703 (US)**

(72) Inventors:
• **MORGEN, Michael**
**Bend, Oregon 97703 (US)**
• **MILLER, Warren**
**Bend, Oregon 97703 (US)**
• **VODAK, David**
**Bend, Oregon 97703 (US)**
• **CAPE, Jonathan**
**Bend, Oregon 97703 (US)**
• **ADAM, Molly**
**Bend, Oregon 97703 (US)**

(74) Representative: **De Clercq & Partners**
**Edgard Gevaertdreef 10a**
**9830 Sint-Martens-Latem (BE)**

(56) References cited:
**WO-A1-2015/138837      WO-A1-2019/220282**
**WO-A2-2007/060384      CN-A- 110 037 990**
**US-A1- 2007 218 012**

**Description**

The invention discloses a method for preparation of spray dried solid dispersions, SDD, comprising an active agent, AA, such as an active pharmaceutical ingredient, API, and a dispersion polymer, DISPPOL, wherein the spray drying is done with a solution of AA and of DISPPOL in a solvent comprising $C_{1-3}$ alkanol and acetic acid, and optionally water.

**BACKGROUND OF THE INVENTION**

Spray dried solid dispersions, SDD, comprising an active pharmaceutical ingredient, API, and a dispersion polymer, DISPPOL, are typically produced by dissolving the dispersion polymer and the API in a volatile solvent, such as methanol or acetone, or in a mixture of solvents, followed by spray drying. In cases where the API has limited solubility, e.g. < 1 wt%, in the spray drying solvent, an API suspension can be heated to a temperature either below or above the solvent's ambient pressure boiling point, this is known as "hot spray drying process", resulting in a higher dissolved concentration of API. In some cases, even the higher temperatures do not give adequate API concentrations that are economical for a spray drying process, or cause other problems such as chemical degradation of the API, or bear the risk of incomplete API dissolution in the heat exchanger. Alternate, non-preferred volatile solvents can provide increased solubility of the API, but these solvents have other disadvantages that make them less desirable, e.g. high cost, toxicity, poor equipment compatibility, poor commercial availability, high disposal costs, challenges removing to sufficiently low levels, higher viscosity.

WO 2019/220282 A1 discloses in Example 1 spray drying of a solution of erlotinib and a dispersion polymer (PMMAMA or hydroxypropyl methylcellulose acetate succinate H grade) in methanol to provide a spray dried dispersion. The presence of an acid in the spray solution is not mentioned.

WO 2007/060384 A2 discloses in example 4 spray-drying of salbutamol sulphate. Example 4 does not disclose the spray solvent, contrary to examples 2 and 3 which refer to example 1. But assuming that the spray solvent of example 1 was also used in example 4, then the spray solvent contained ca. 550 ml + 33 ml = 583 ml water and 30 ml EtOH, that is 95 wt% water. Since the sulphate of salbutamol was used for spray drying, the free base form of the drug is not obtained after spray drying, but again the sulphate.

Also example 11 discloses spray-drying of salbutamol sulphate, so again the sulphate is contained in the SDD, not its free base form. 1 ml + ca. 49 ml = 50 ml water and ca. 21 ml, that are ca. 17 g of EtOH, assuming the described dilution to 100 ml with aqueous ethanol (30% v/v) needed ca. 70 ml of the aqueous ethanol. So the respective total content of water in the w/o/w emulsion was ca. 75 wt% water based on the combined amount of water and ethanol.

CN110037990A discloses solid amorphous dispersions of apixaban. Apixaban has two pKa values, one acidic pKa of ca. 13 at which half of the acidic site is deprotonated, and a basic pKa of < 2 at which half of the respective basic site is protonated (source: https://go.drugbank.com/drugs/DB06605). A combination of THF and acetic acid is used as solvent, ethanol is mentioned only for providing low solubility for apixaban.

WO 2015/138837 A1 discloses amorphous solid dispersions of ivosidenib. Ivosidenib has a basic pKa of ca. 1.81 (https://go.drugbank.com/drugs/DB14568). Only one example of spray drying is disclosed, no acid was contained in the spray mixture.

US 2007/0218012 A1 discloses in the examples methylene chloride and acetone as spray solvents for preparing sold dispersion of VX-950, Telaprevir, which has a basic pKa of -0.69 (https://go.drugbank.com/drugs/DB05521).

WO 2013/105894 A1 discloses the preparation of stable, amorphous hybrid nanoparticles with various protein kinase inhibitors PKI. Solvents used are DMSO, acetone and trifluoroethanol (TFE). A solution of PKI and polymer in said solvents is pumped through XSpray's RightSize nozzle together with a $CO_2$ stream, which method is different from conventional spray drying of a spray solution. Neither $C_{1-3}$ alkanols nor acetic acids are mentioned.

There was a need for a method for preparing spray dried solid dispersion of an active agent, AA, which is a weak organic base in its free base form, and dispersion polymers, which allows for dissolving the APIs in easily processable spray drying solvents such as $C_{1-3}$ alkanols at modest temperature, i.e. a temperature below the ambient pressure boiling point, at sufficiently high concentrations to enable economical throughput of SDDs. By the method the free base should be obtained as a SDD.

It was found that acetic acid may be used as processing aid in such spray drying method. The solubility of the AA is increased, which allows for higher concentration of AA in the spray solution than in absence of acetic acid. Increased AA solubility gives higher manufacturing throughput, and potentially better spray dried particle characteristics than what is achievable with lower solids content spray solutions. When acetic acid is used not only as a processing aid, but as the only solvent then viscosities tend to be high.

**Abbreviations and definitions used in this specification**

| | |
|---|---|
| AA | active agent |
| API | active pharmaceutical ingredient |
| ASD | amorphous solid dispersion |
| dasatinib | CAS 302962-49-8, basic pKa 7.2 |
| DISPPOL | dispersion polymer |
| eq | equivalents |
| gefitinib | crystalline gefitinib, CAS 184475-35-2, basic pKa = 6.8 |
| glacial acetic acid | water-free (anhydrous) acetic acid, acetic acid 100% |
| HPMCAS | Hydroxypropyl Methylcellulose Acetate Succinate, Hypromellose Acetate Succinate, CAS 71138-97-1 |
| pKa | the pKa of a basic site of an organic Bronstedt base is the pH at which half of these basic sites are protonated. At a pH which is lower than this basic pKa more than half of these basic sites are protonated, that is ionized. This pKa of a basic site is also called basic pKa. In contrast thereof the pKa of an acidic site of an organic Bronstedt acid is the pH at which half of these acidic sites are deprotonated, that is ionized. At a pH which is higher than this acidic pKa more than half of these acidic sites are deprotonated. This pKa of an acidic site is also called acidic pKa. pKa values are available in the internet, they may also be calculated, for example by ADMET predictor® software, Simulations Plus, Inc. (Nasdaq: SLP) or measured in the lab. |
| PVP-VA | Vinylpyrrolidone-vinyl acetate copolymer |
| SDD | Spray dried solid dispersions |
| SPRAYDRY | method for preparing a spray dried solid dispersion SDD |
| SPRAYSOL | spray solution |
| wt% | weight % |

## SUMMARY OF THE INVENTION

Subject of the invention is a method SPRAYDRY for preparing a spray dried solid dispersion, SDD, of an active agent, AA, which is an organic Bronstedt base, comprising:

> a. combining an active agent, AA, a dispersion polymer, DISPPOL, acetic acid, and a solvent, SOLV, to form a spray solution, SPRAYSOL, wherein
>
>> i. SOLV comprises a $C_{1-3}$ alkanol,
>> the amount of the $C_{1-3}$ alkanol in SOLV is at least 50 wt%, with the wt% being based on the weight of SOLV;
>> ii. AA is in its free base form when combined with the acetic acid and SOLV to form SPRAYSOL, and, in its free base form, has a basic pKa of 4 or greater, and AA has a solubility of 40 mg/mL or less in SOLV,
>> iii. SPRAYSOL is not a supersaturated solution of AA in SOLV and acetic acid;
>
> b. spray drying SPRAYSOL to form a SDD comprising AA and DISPPOL; SPRAYSOL has only one liquid phase.

## DESCRIPTION OF THE DRAWINGS

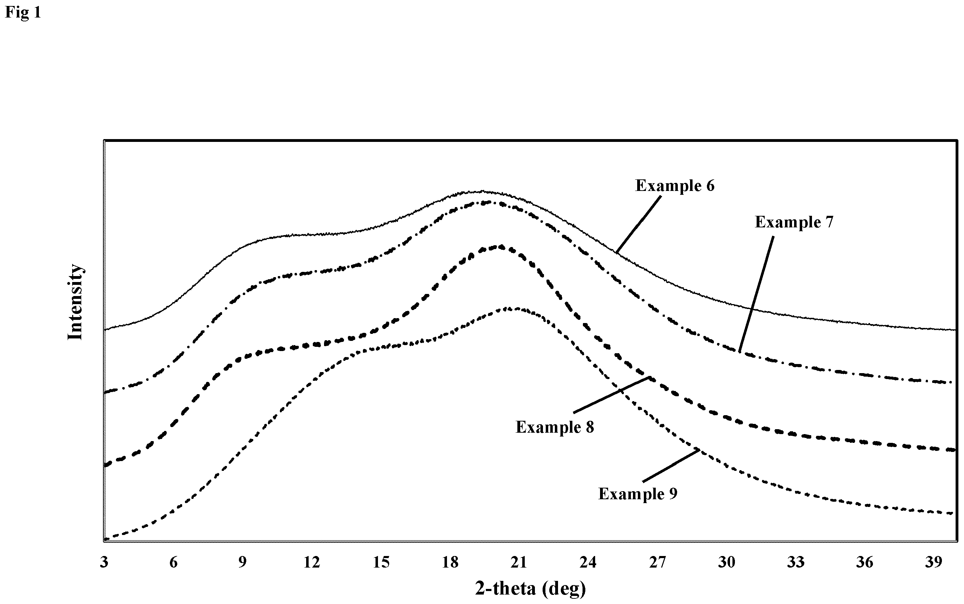

**Figure 1:** PXRD patterns for Examples 6 to 9 showing the amorphous nature of the samples.

## DETAILED DESCRIPTION OF THE INVENTION

SDD is a spray dried solid dispersion of AA in DISPPOL. AA and DISPPOL are preferably homogeneously mixed in SDD.

In a solid dispersion of AA in DISPPOL, AA may be homogeneously and preferably also molecularly dispersed in DISPPOL. AA and DISPPOL may form a solid solution in SDD. AA may be amorphous or substantially amorphous in SDD; substantially means that at least 80 wt%, preferably at least 90 wt%, more preferably at least 95 wt%, even more preferably at least 98 wt%, especially at least 99% wt%, of AA is amorphous; the wt% being based on the total weight of AA in SDD. SDD therefore may be an amorphous SDD. The amorphous nature of AA may be evidenced by a lack of sharp Bragg diffraction peaks in the x-ray pattern when SDD is analyzed by a powder X-Ray Diffraction, PXRD.

Possible parameters and settings for a x-ray diffractometer are equipment with a Cu-Kalpha source, setting in modified parallel beam geometry between 3 and 40° 2Theta and a scan rate of 2°/min with a 0.0° step size. Another evidence for the amorphous nature of AA in the SDD may be a single glass transition temperature, Tg. A single Tg is also evidence of a homogeneous mixture of amorphous AA and polymer. Samples as such without any further sample preparation may be used for the determination of the Tg, the determination may run for example in modulated mode at a scan rate of 2.5 °C/min, modulation of $\pm$ 1.5 °C/min, and a scan range from 0 to 180 °C. Amorphous nature of AA shows a Tg which is equal to the Tg of neat DSISPPOL or which is between the Tg of the polymer and the Tg of the AA. The Tg of the SDD is often similar to the weighted average of the Tg of AA and the Tg of DISPPOL. SDD is amorphous or substantially, SDD can also be called ASD.

SPRAYSOL is a stable solution of AA in SOLV and acetic acid.

SPRAYSOL has only one liquid phase. This means that the liquid phase of SPRAYSOL has not more than one liquid phase but is has only one liquid phase, so for example it has not 2 or 3 separate liquid phases, which would be the case if a liquid phase would be a water/oil or water/oil/water emulsion or the like.

The amount of AA with respect to SOLV is above the solubility of AA in SOLV in absence of acetic acid.

Possible amount of AA in SPRAYSOL may be at least 0.5 wt%, preferably at least 1 wt%, more preferably at least 3 wt%, with the wt% being based on the weight of SPRAYSOL.

Possible amount of AA may be up to 10 wt%, preferably up to 7.5 wt%, more preferably up to 5 wt%.

Any of the lower limits may be combined with any of the upper limits of AA in SPRAYSOL.

For example, possible amounts of AA in SPRAYSOL may be from 0.5 wt% to 10 wt%, preferably from 1 wt% to 10 wt%, more preferably from 3 wt% to 10 wt%, with the wt% being based on the weight of SPRAYSOL.

The amount of acetic acid is sufficient to solubilize AA in SOLV.

The amount of acetic acid may be 1 to 50 eq, preferably 1 to 40 eq, more preferably 1 to 30 eq, even more preferably 1 to 25 eq, based on the molar amount of AA.

The amount of acetic acid may be up to 50 wt%, preferably up to 40 wt%, more preferably up to 30 wt%, even more preferably up to 25 wt%, especially up to 15 wt%, more especially up to 10 wt%, even more especially up to 7.5, in particular up to 5 wt%, the wt% being based on the weight of SOLV.

The amount of acetic acid may be from 0.05 to 50 wt%, preferably from 0.05 to 40 wt%, more preferably from 0.05 to 30 wt%, even more preferably from 0.05 to 25 wt%, especially from 0.05 to 15 wt%, more especially from 0.1 to 10 wt%, even more especially from 0.1 to 7.5, in particular from 0.1 to 5 wt%, the wt% being based on the weight of SOLV.

The $C_{1-3}$ alkanol of SOLV may be methanol, ethanol or isopropanol, preferably methanol or ethanol, more preferably methanol.

The amount of the $C_{1-3}$ alkanol in SOLV may be at least 60 wt%, or at least 65 wt%, or at least 67.5 wt%, or at least 70 wt%, or at least 75 wt%, or at least 80 wt%, or at least 85 wt%, or at least 90 wt%, or at least 95 wt%; with the wt% being based on the weight of SOLV.

In another embodiment, SOLV may further comprise water.

When SOLV comprises water, then SOLV comprises not more than 40 wt%, preferably not more than 35 wt%, more preferably not more than 32.5 wt%, even more preferably not more than 30 wt%, especially not more than 25 wt%, more especially not more than 20 wt%, of water, with the wt% being based on the combined weights of $C_{1-3}$ alkanol and water.

When SOLV comprises water, then the weight ratio $C_{1-3}$ alkanol : water in SOLV may be from 99 : 1 to 60 : 40, preferably from 99 : 1 to 65 : 35, more preferably from 99 : 1 to 67.5 : 32.5, even more preferably from 99 : 1 to 70 : 30, especially from 99 : 1 to 75 : 25, more especially from 99 : 1 to 80 : 20.

In one embodiment, SOLV consists of $C_{1-3}$ alkanol and water.

In one embodiment, SOLV consists of $C_{1-3}$ alkanol.

AA is in its free base form when combined with or added to the acetic acid and SOLV to form SPRAYSOL.

AA may be present in SPRAYSOL in its free base form, in its protonated form or in both forms, depending on its basic pKa.

The SDD may comprise from 1 to 99 wt%, preferably from 10 to 95 wt%, more preferably from 10 to 80 wt%, even more preferably from 20 to 60 wt%, of AA, the wt% being based on the weight of the SDD.

The SDD may comprise from 1 to 99 wt%, preferably from 20 to 90 wt%, more preferably from 40 to 80 wt%, of DISPPOL, the wt% being based on the weight of the SDD.

Preferably, the combined content of AA and DISPPOL in SDD is from 65 to 100 wt%, more preferably from 67.5 to 100 wt%, even more preferably from 80 to 100 wt%; especially from 90 to 100 wt%; more especially from 95 to 100 wt%;

the wt% being based on the weight of the SDD;
in one embodiment, the SDD consists of AA and DISPPOL.

Relative amounts of AA to DISPPOL in SDD may be from 50 : 1 to 1 : 50, preferably from 25 : 1 to 1 : 25, more preferably from 10 : 1 to 1 : 10 (w/w).

Amounts of DISPPOL and of AA in SPRAYSOL are chosen such that a predefined amount of DISPPOL and of AA in SDD provided.

DISPPOL is present in SPRAYSOL in a dissolved state, the amounts of DISPPOL and SOLV are chosen respectively.

For example amounts of DISPPOL in SPRAYSOL may be from 0.5 wt% to 20 wt%, preferably from 1 wt% to 20 wt%, more preferably from 2.5 wt% to 15 wt%, even more preferably from 5 wt% to 10 wt%, with the wt% being based on the weight of SPRAYSOL.

Preferably, AA may have a solubility of 30 mg/mL or less, more preferably of 20 mg/mL or less, even more preferably of 10 mg/mL or less, in SOLV.

AA is an organic Bronstedt base.

AA may be a biologically active compound. The biologically active compound may be desired to be administered to a patient in need of AA.

AA may be a drug, medicament, pharmaceutical, therapeutic agent, nutraceutical, an active pharmaceutical ingredient, API.

AA may be a "small molecule," generally having a molecular weight of 2000 Daltons or less. An API, that is AA, may be dasatinib or gefitinib.

AA may be one or more AAs; SDD may contain one or more AAs.

In SPRAYDRY both SOLV and the acetic acid are evaporated. AA is obtained in the SDD in its free base form.

SPRAYDRY recovers and provides AA in its free base form, so essentially all of AA is present in the SDD in its free base form, this means that the basic site of AA which has said basic pKa of 4 or greater is essentially present in deprotonated state in the SDD.

Preferably, AA has a basic pKa of 5 or greater.

DISPPOL may comprise one or more dispersion polymers, preferably 1, 2, 3 or 4, more preferably 1, 2 or 3, even more preferably 1 or 2 dispersion polymers.

DISPPOL may be a pharmaceutically acceptable dispersion polymer.

Suitable DISPPOL include, but are not limited to, hydroxypropyl methylcellulose acetate succinate (HPMCAS), hydroxypropyl methylcellulose phthalate (HPMCP), hydroxypropyl methyl cellulose (HPMC), hydroxypropyl cellulose (HPC), cellulose acetate phthalate (CAP), carboxymethyl ethyl cellulose (CMEC), polyvinylpyrrolidone (PVP), poly(vinylpyrrolidone-co-vinyl acetate) (PVP-VA), poly(methacrylic acid-co-methyl methacrylate) (PMMAMAA), poly(methacrylic acid-co-ethyl acrylate), or any combination thereof.

Suitable PMMAMAA polymers include, but are not limited to, poly(methacrylic acid-co-methyl methacrylate) 1:1 (for example Eudragit® L100), and poly(methacrylic acid-co-methyl methacrylate) 1:2 (for example Eudragit® S100). Eudragit® are polymer products of Evonik Industries AG, 45128 Essen, Germany.

The poly(methacrylic acid-co-ethyl acrylate) may be poly(methacrylic acid-co-ethyl acrylate) 1:1.

In some embodiments, DISPPOL is HPMCAS or PMMAMAA.

SPRAYSOL may be fed into the spray dryer with a temperature of SPRAYSOL up to the boiling point of SPRAYSOL at ambient pressure; preferably with a temperature of from 4 °C to the boiling point of SPRAYSOL at ambient pressure, preferably from 4 °C to a temperature below the boiling point of SPRAYSOL at ambient pressure, more preferably from room temperature to 60°C. In the context of this invention the term "SPRAYSOL may be fed into the spray dryer with a temperature of SPRAYSOL" means that "SPRAYSOL is spray dried with a temperature of SPRAYSOL".

The spray drying may be done with an inlet temperature of from 60 to 165 °C.

The spray drying may be done with an outlet temperature equal to or less than the boiling point of SOLV.

The spray drying may be done with any inert gas commonly used for spray drying, such as nitrogen.

The spray drying does preferably not use $CO_2$ for dissolving or solubilizing AA. $CO_2$ is preferably not added to or mixed with SPRAYSOL. $CO_2$ is preferably not mixed with SPRAYSOL in the nozzle of the spray dryer. When $CO_2$ is used in the spray drying then it may be used in the conventional way as conventional inert drying gas in the spray drying process.

SPRAYSOL may further comprises a dissolved surfactant SURF.

SURF may be mixed with SPRAYSOL.

SURF may be for example a fatty acid and alkyl sulfonate, docusate sodium (for example available from Mallinckrodt Spec. Chem., St. Louis, Mo.), polyoxyethylene sorbitan fatty acid esters (for example Tween®, available from ICI Americas Inc, Wilmington, Del., or Liposorb® P-20, available from Lipochem Inc, Patterson, N.J., or Capmul® POE-0, available from Abitec Corp., Janesville, Wis.), natural surfactants such as sodium taurocholic acid, 1-palmitoyl-2-oleoyl-sn-glycero-3-phosphocholine, lecithin, other phospholipids and mono- and diglycerides, vitamin E TPGS, PEO-PPO-PEO triblock copolymers (for example known under the tradename pluronics), or PEO (PEO are also called PEG, polyethyleneglycols (PEG)).

The amount of SURF may be up to 10 wt%, the wt% being based on the weight of SDD.

SPRAYSOL may further comprises pharmaceutically acceptable excipients, such as fillers, disintegrating agents, pigments, binders, lubricants, flavorants, and so forth which can be used for customary purposes and in typical amounts known to the person skilled on the art.

The SDD may comprise residual acetic acid, preferably in low amounts; the content of residual acetic acid in SDD may be 5'000 ppm or less, preferably 500 ppm or less, more preferably of 100 ppm or less, the ppm being based on the weight of SDD.

Also after the spraying any content of residual acetic acid in SDD may be lowered to a predefined content of residual acetic acid, this may be done with an additional drying step after spray drying.

The SDD may comprise residual SOLV, the content of residual SOLV in SDD may be 5'000 ppm or less, preferably 3'000 ppm or less, more preferably 500 ppm or less, even more preferably of 100 ppm or less, the ppm being based on the weight of SDD.

Also after the spraying any content of residual SOLV in SDD may be lowered to a predefined content of residual SOLV in SD.

Any residual content of acetic acid or of SOLV in SDD may be reduced to the desired predefined and final content by submitting SDD after the spray drying to a second drying. Secondary drying may be done using a tray dryer or any agitated dryer known to the skilled person for drying solids.

Further subject of the invention is a spray dried solid dispersion, SDD, wherein the SDD is obtainable by the method SPRAYDRY;

with SDD and SPRAYDRY as defined herein, also with all their embodiments.

## EXAMPLES

### Materials and abbreviations

| | |
|---|---|
| dasatinib | crystalline Dasatinib free base, LC Laboratories, Woburn, MA, USA, >99+% |
| gefitinib | gefitinib free base |
| GC | gas chromatography |
| HPMCAS-MG | AquaSolve, Ashland, pharma grade |
| HPMC E3 | Methocel E3, Hydroxypropyl Methylcellulose, Manufacturer DuPont de Nemours, Inc., Wilmington, Delaware, USA |
| PMMAMAA | Eudragit L-100, Evonik, pharma grade. |
| PVP-VA64 | PVP-VA, Kollidon VA64 from BASF, Ludwigshafen, Germany |
| PXRD | powder x-ray diffraction |
| RCF | relative centrifugal force |
| RH | relative humidity |
| TGA | thermo gravimetric analysis |

### Example 1: Solubility of dasatinib in methanol

Dasatinib free base was recrystallized from methanol and dried. Crystalline dasatinib was added in excess to methanol to form a saturated solution at 25 °C. The solution was analyzed by TGA and found to contain 3.1 mg/mL dasatinib.

### Example 2: Increasing dasatinib concentration dissolved in methanol with acetic aid

200 mg of crystalline dasatinib was slurried in 10 mL of methanol at 25 °C. The slurry was titrated with glacial acetic in 100 microliter increments until the dasatinib was completely dissolved. After a total of 500 microliter (21.5 eq based on the molar amount of dasatinib) of glacial acetic acid was added the mixture became clear. The concentration of dissolved dasatinib was approximately 19 mg/mL and ca. 6 fold higher than in methanol without acetic acid that was used in example 1.

### Example 3: 25% dasatinib/HPMCAS-MG SDD

HPMCAS-MG polymer, 7.51 g, was dissolved in 98.2 g of methanol at 19 °C. Crystalline dasatinib free base, 2.50 g was added forming a slurry. Glacial acetic acid, 6.5 g (21 eq based on the molar amount of dasatinib), was added with stirring and placed in a 25 °C water bath for 1 hour converting the slurry into a solution with 8.7 wt% dissolved solids. The solution was removed from the water bath, allowed to cool for 30 min to 19 °C and then spray-dried. The solution did not contain dasatinib in solid form, instead it contained the dasatinib in a completely dissolved state, and it had only one liquid phase.

The solution was spray dried using a custom built lab-scale spray dryer. The solution was pumped into a lab-scale 0.3 m diameter stainless steel spray drying chamber using a peristaltic pump. The flow rate of the solution was 20 g/min., atomization was done through a two-fluid nozzle ¼ J series with an 1650 liquid body and 64 air cap made by Spraying Systems Company, Glendale Heights, Il, 60187-7901, US. Sheath gas was used to atomize the solution at a pressure of 15 psi. Heated nitrogen gas was introduced into the spray chamber at a temperature of 115 to 120 °C and flow rate of 500 g/min. The outlet temperature of the gas exiting the chamber was 45 to 50 °C. The resulting SDD was collected using a cyclone to separate the solid particles from the gas stream.

Residual acetic acid was removed by drying on a tray dryer at 60 °C and 30% RH for 8 h. The residual acetic acid was measured to be 230 ppm by GC. The residual methanol was measured to be below 100 ppm by GC. PXRD shows a homogeneous amorphous solid dispersion.

**Example 4: 25% dasatinib/ PMMAMAA SDD**

Eudragit L-100 (PMMAMAA) polymer, 7.50 g, was dissolved in 98.4 g of methanol at 19 °C. Crystalline dasatinib free base, 2.51 g, was added forming a slurry. Glacial acetic acid, 6.5 g (21 eq based on the molar amount of dasatinib), was added with stirring and placed in a 25 °C water bath for 30 min converting the slurry into a solution with 8.7 wt% dissolved solids. The solution was removed from the water bath, allowed to cool for 30 min to 19 °C and then spray-dried.

The solution did not contain dasatinib in solid form, instead it contained the dasatinib in a completely dissolved state, and it had only one liquid phase.

The solution was spray dried using a custom built lab-scale spray dryer. The solution was pumped into a lab-scale 0.3 m diameter stainless steel spray drying chamber using a peristaltic pump. The flow rate of the solution was 20 g/min., atomization was done through a two-fluid nozzle ¼ J series with an 1650 liquid body and 64 air cap made by Spraying Systems Company, Glendale Heights, IL 60187-7901, United States. Sheath gas was used to atomize the solution at a pressure of 15 psi. Heated nitrogen gas was introduced into the spray chamber at a temperature of 115 to 120 °C and flow rate of 500 g/min. The outlet temperature of the gas exiting the chamber was 45 to 50 °C. The resulting SDD was collected using a cyclone to separate the solid particles from the gas stream.

Residual acetic acid was removed by drying on a tray dryer at 60 °C and 30% RH for 24 h. The residual acetic acid was measured to be 5'000 ppm by GC. The residual methanol was measured to be below 100 ppm by GC. PXRD shows a homogeneous amorphous solid dispersion.

**Example 5: Gefitinib solubilities**

Crystalline gefitinib was added in excess to methanol and methanol:water mixtures to form saturated solutions at 20°C. After 24 hour of stirring, 1 mL aliquots were centrifuged at 10'000 RCF for 3 min. The supernatant was then analyzed for gefitinib concentration by HPLC.

The solubility of gefitinib in solvent mixtures with acetic acid were obtained by suspending 300 mg of crystalline gefitinib in 5 mL of solvent (methanol:water) containing 150 microliters of acetic acid at 20 °C (3.9 eq of acetic acid). With the exception of 100% methanol, all solutions were visually soluble at 60 mg/mL. The solubility in 100% methanol with acetic acid was determined after separating undissolved solids by centrifugation of a 1 mL aliquot at 10'000 RCF for 3 min after 1 hour of stirring. The supernatant was then analyzed for gefitinib concentration by HPLC.

Table 1 shows the gefitinib solubility enhancement in solvent mixtures using 3.9 eq of acetic acid; the enhancement is expressed in Table 1 in form of an Enhancement Factor which is the ratio of

$$[\text{Solubility with acetic acid}] / [\text{Solubility without acetic acid}]$$

| Table 1 | | | |
|---|---|---|---|
| Solvent composition Methanol:$H_2O$ | Solubility without acetic acid [mg/mL] | Solubility with acetic acid (*) [mg/mL] | Enhancement factor |
| 100:0 | 5.2 | 31 | 5.9 |

(continued)

| Table 1 | | | |
|---|---|---|---|
| Solvent composition Methanol:$H_2O$ | Solubility without acetic acid [mg/mL] | Solubility with acetic acid (*) [mg/mL] | Enhancement factor |
| 90:10 | 6.0 | >60 | >10 |
| 80:20 | 6.3 | >60 | >10 |
| 70:30 | 4.0 | >60 | >15 |
| 60:40 | 2.1 | >60 | >29 |
| (*) 3.9 molar equivalents of acetic acid added | | | |

Table 2 shows the spray solution compositions for examples 6 to 9

| Table 2 | | | | | | |
|---|---|---|---|---|---|---|
| Example | Polymer | Solvent composition [wt% based on weight of solvent] | | | | Dissolved solids concentration |
| | | methanol | | water | acetic acid | [wt% based on weight of solution] |
| 6 | HPMCAS-MG | 84.5 | | 14.8 | 0.7 | 8.70 |
| 7 | HPMCAS-MG | 69.6 | | 29.7 | 0.7 | 8.36 |
| 8 | HPMC E3 | 79.5 | | 19.9 | 0.6 | 8.61 |
| 9 | PVP-VA64 | 84.6 | | 14.7 | 0.7 | 8.69 |

**Example 6: 25:75 gefitinib:HPMCAS-MG SDD (85:15 MeOH:water)**

A slurry was made by first dissolving 9 g of HPMCAS-MG in a mixture of 98 g of methanol and 18.7 g water at 22 °C, and then adding 3 g of crystalline gefitinib to form a drug slurry. To this slurry was added 11 mL of 0.075 g/mL glacial acetic acid in methanol. The mixture was stirred for at least 30 min to dissolve the drug and thereby to form a solution SPRAYSOLV.

SPRAYSOLV did not contain gefitinib in solid form, instead it contained the gefitinib in a completely dissolved state, and it had only one liquid phase.

The solution was spray dried using a custom built spray dryer. The solution was pumped into a lab-scale 0.3 m diameter stainless steel spray drying chamber using a peristaltic pump at a solution flow rate of 15 g/min. The spray solution was atomized using a two-fluid ¼ J series nozzle with a 1650 liquid body and 64 air cap (Spraying Systems Company, Glendale Heights, IL 60187-7901, US). Room temperature sheath gas (15 to 20 psi) was used to atomize the solution and heated nitrogen gas (115 to 125 °C inlet, 45 to 50 °C outlet, 500 g/min) was used to dry the particles. The resulting SDD was collected using a cyclone to separate the solid particles from the gas stream.

Samples were placed in a tray dryer at two conditions: 40 °C / 15% RH and 60 °C / 30% RH. Methanol was measured by GC to be <100 ppm after 1 h at both conditions, and acetic acid was measured to be <500 ppm after 8 h at 40 °C / 15% RH and after 1 h at 60 °C / 30% RH. PXRD showed the spray dried material to be amorphous.

**Example 7: 25:75 gefitinib:HPMCAS-MG SDD (70:30 MeOH:water)**

A slurry was made by first dissolving 9 g of HPMCAS-MG in a mixture of 83.1 g of methanol and 39.1 g water at 22 °C, and then adding 3 g of crystalline gefitinib to form a drug slurry. To this slurry was added 11 mL of 0.075 g/mL glacial acetic acid in methanol. The mixture was stirred for at least 30 minutes to dissolve the drug. The mixture was stirred for at least 30 min to dissolve the drug and thereby to form a solution SPRAYSOLV. SPRAYSOLV did not contain gefitinib in solid form, instead it contained the gefitinib in a completely dissolved state, and it had only one liquid phase.

The solution was spray dried using the procedure of Example 6.

Samples were placed in a tray dryer at two conditions: 40 °C / 15% RH and 60 °C / 30% RH. Methanol was measured by GC to be <100 ppm after 1 h at both conditions, and acetic acid is measured to be <500 ppm at after 8 h at 40 °C / 15% RH and after 1 hour at 60 °C / 30% RH. PXRD showed the spray dried material to be amorphous.

**Example 8: 25:75 gefitinib:HPMC E3 SDD**

A slurry was made by first dissolving 9 g of HPMC E3 in a mixture of 51.1 g of methanol and 25.3 g water at 22 °C, adding an additional 42.3 g methanol after the HPMC E3 was dissolved, then adding 3 g of crystalline gefitinib. To this slurry was added 10 mL of 0.075 g/mL glacial acetic acid in methanol. The mixture was stirred for at least 30 min to dissolve the drug and thereby to form a solution SPRAYSOLV.
SPRAYSOLV did not contain gefitinib in solid form, instead it contained the gefitinib in a completely dissolved state, and it had only one liquid phase.
The solution was spray dried using the procedure of Example 6.
Samples were placed in a tray dryer at two conditions: 40 °C / 15% RH and 60 °C / 30% RH. Methanol was measured by GC to be <100 ppm after 1 h at both conditions, and acetic acid is measured to be <500 ppm at after 24 h at 40 °C / 15% RH and after 2 h at 60 °C / 30% RH. PXRD showed the spray dried material to be amorphous.

**Example 9: 25:75 gefitinib:PVP-VA64 SDD**

A slurry was made by first dissolving 9 g of PVP-VA64 in a mixture of 98 g of methanol and 18.6 g water at 22 °C, and then adding 3 g of crystalline gefitinib to form a drug slurry. To this slurry was added 11 mL of 0.075 g/mL glacial acetic acid in methanol. The mixture was stirred for at least 30 min to dissolve the drug and thereby to form a solution SPRAYSOLV. SPRAYSOLV did not contain gefitinib in solid form, instead it contained the gefitinib in a completely dissolved state, and it had only one liquid phase.
The solution was spray dried using the procedure of Example 6.
Samples were placed in a tray dryer at two conditions: 40 °C / 15% RH and 60 °C / 30% RH. Methanol was measured by GC to be <100 ppm after 1 h at both conditions, and acetic acid is measured to be <500 ppm at after 24 h at 40 °C / 15% RH and after 4 h at 60 °C / 30% RH. PXRD showed the spray dried material to be amorphous.
Figure 1 shows the PXRD patterns for Examples 6 to 9, tray dried for 24 h at 40 °C / 15% RH. The lack of sharp diffraction peaks suggests the drug is amorphous. SDDs dried at 60 °C / 30% RH showed similar PXRD patterns.

**Claims**

1. A method for preparing a spray dried solid dispersion of an active agent, which is an organic Bronstedt base, comprising:

   a. combining an active agent, a dispersion polymer, acetic acid, and a solvent, to form a spray solution, wherein

   i. the solvent comprises a $C_{1-3}$ alkanol,
   the amount of the $C_{1-3}$ alkanol in the solvent is at least 50 wt%, with the wt% being based on the weight of the solvent;
   ii. the active agent is in its free base form when combined with the acetic acid and the solvent to form the spray solution, and, in its free base form, has a basic pKa of 4 or greater, and the active agent has a solubility of 40 mg/mL or less in the solvent,
   iii. the spray solution is not a supersaturated solution of the active agent in the solvent and acetic acid;

   b. spray drying the spray solutionto form a spray dried solid dispersion comprising the active agent and the dispersion polymer;

   wherein the spray solution has only one liquid phase.

2. The method according to claim 1, wherein
   the amount of active agent with respect to solvent is above the solubility of said active agent in the solvent in absence of acetic acid.

3. The method according to claim 1 or 2, wherein
   the amount of active agent in the spray solution is at least 0.5 wt%, with the wt% being based on the weight of the spray solution.

4.  The method according to one or more of claims 1 to 3, wherein
    the amount of acetic acid is 1 to 50 eq based on the molar amount of active agent.

5.  The method according to one or more of claims 1 to 3, wherein
    the amount of acetic acid is from 0.05 to 50 wt%, the wt% being based on the weight of the solvent.

6.  The method according to one or more of claims 1 to 5, wherein
    the solvent is methanol, ethanol or isopropanol.

7.  The method according to one or more of claims 1 to 6, wherein
    the solvent further comprises water.

8.  The method according to claim 7, wherein
    the weight ratio $C_{1-3}$ alkanol : water in the solvent may be from 99 : 1 to 60 : 40.

9.  The method according to one or more of claims 1 to 8, wherein
    the spray dried solid dispersion comprises from 1 to 99 wt% of active agent, the wt% being based on the weight of the spray dried solid dispersion.

10. The method according to one or more of claims 1 to 9, wherein
    the spray dried solid dispersion comprises from 1 to 99 wt% of the dispersion polymer, the wt% being based on the weight of the spray dried solid dispersion.

11. The method according to one or more of claims 1 to 10, wherein
    the combined content of active agent and dispersion polymer in the spray dried solid dispersion is from 65 to 100 wt%, the wt% being based on the weight of the spray dried solid dispersion.

12. The method according to one or more of claims 1 to 11, wherein the
    active agent is a biologically active compound.

13. The method according to one or more of claims 1 to 12, wherein
    the active agent is a drug, medicament, pharmaceutical, therapeutic agent, nutraceutical, an active pharmaceutical ingredient.

14. The method according to one or more of claims 1 to 13, wherein
    the active agent has a basic pKa of 5 or greater.

15. The method according to one or more of claims 1 to 14, wherein
    the dispersion polymercomprises one or more dispersion polymers.

16. The method according to one or more of claims 1 to 15, wherein
    the dispersion polymeris a pharmaceutically acceptable dispersion polymer.

17. The method according to one or more of claims 1 to 16, wherein
    the dispersion polymeris hydroxypropyl methylcellulose acetate succinate, hydroxypropyl methylcellulose phthalate, hydroxypropyl methyl cellulose, hydroxypropyl cellulose, cellulose acetate phthalate, carboxymethyl ethyl cellulose, polyvinylpyrrolidone, poly(vinylpyrrolidone-co-vinyl acetate), poly(methacrylic acid-co-methyl methacrylate), poly(methacrylic acid-co-ethyl acrylate), or any combination thereof.

18. The method according to one or more of claims 1 to 17, wherein
    the dispersion polymeris HPMCAS or PMMAMAA.

19. The method according to one or more of claims 1 to 18, wherein
    the SDD comprises residual acetic acid; the content of residual acetic acid in the spray dried solid dispersion is 5'000 ppm or less, the ppm being based on the weight of the spray dried solid dispersion.

20. The method according to one or more of claims 1 to 19, wherein
the SDD comprises residual solvent, the content of residual solvent in the spray dried solid dispersion is 5'000 ppm or less, the ppm being based on the weight of the spray dried solid dispersion.

21. The method according to one or more of claims 1 to 6 and 9 to 20, wherein
the solvent consists of $C_{1-3}$ alkanol.

22. The method according to one or more of claims 1 to 21, wherein
the solvent consists of $C_{1-3}$ alkanol and water.

23. A spray dried solid dispersion obtainable by the method according to any one of claims 1 to 22.

**Patentansprüche**

1. Verfahren zum Herstellen einer sprühgetrockneten Feststoffdispersion eines Wirkstoffs, bei dem es sich um eine organische Bronstedt-Base handelt, umfassend:

a. Kombinieren eines Wirkstoffs, eines Dispersionspolymers, Essigsäure und eines Lösungsmittels, um eine Sprühlösung zu bilden, wobei

i. das Lösungsmittel ein $C_{1-3}$-Alkanol umfasst,
die Menge an $C_{1-3}$-Alkanol in dem Lösungsmittel mindestens 50 Gew.-% beträgt, wobei die Gew.-% auf das Gewicht des Lösungsmittels bezogen sind;
ii. der Wirkstoff in Form seiner freien Base vorliegt, wenn er mit der Essigsäure und dem Lösungsmittel zum Bilden der Sprühlösung kombiniert wird, und in Form seiner freien Base einen basischen pKa-Wert von 4 oder höher aufweist und der Wirkstoff eine Löslichkeit von 40 mg/ml oder weniger in dem Lösungsmittel aufweist,
iii. die Sprühlösung keine übersättigte Lösung des Wirkstoffs in dem Lösungsmittel und der Essigsäure ist;

b. Sprühtrocknen der Sprühlösung, um eine sprühgetrocknete Feststoffdispersion zu bilden, die den Wirkstoff und das Dispersionspolymer umfasst;

wobei die Sprühlösung nur eine flüssige Phase aufweist.

2. Verfahren nach Anspruch 1, wobei
die Menge an Wirkstoff in Bezug auf das Lösungsmittel ohne Essigsäure über der Löslichkeit des Wirkstoffs in dem Lösungsmittel liegt.

3. Verfahren nach Anspruch 1 oder 2, wobei
die Menge an Wirkstoff in der Sprühlösung mindestens 0,5 Gew.-% beträgt, wobei die Gew.-% auf das Gewicht der Sprühlösung bezogen sind.

4. Verfahren nach einem oder mehreren der Ansprüche 1 bis 3, wobei
die Menge an Essigsäure 1 bis 50 Äq. beträgt, bezogen auf die molare Menge an Wirkstoff.

5. Verfahren nach einem oder mehreren der Ansprüche 1 bis 3, wobei
die Menge an Essigsäure 0,05 bis 50 Gew.-% beträgt, wobei die Gew.-% auf das Gewicht des Lösungsmittels bezogen sind.

6. Verfahren nach einem oder mehreren der Ansprüche 1 bis 5, wobei
das Lösungsmittel Methanol, Ethanol oder Isopropanol ist.

7. Verfahren nach einem oder mehreren der Ansprüche 1 bis 6, wobei
das Lösungsmittel ferner Wasser umfasst.

8. Verfahren nach Anspruch 7, wobei
das Gewichtsverhältnis $C_{1-3}$-Alkanol : Wasser in dem Lösungsmittel 99 : 1 bis 60 : 40 betragen kann.

**9.** Verfahren nach einem oder mehreren der Ansprüche 1 bis 8, wobei die sprühgetrocknete Feststoffdispersion 1 bis 99 Gew.-% Wirkstoff umfasst, wobei die Gew.-% auf das Gewicht der sprühgetrockneten Feststoffdispersion bezogen sind.

**10.** Verfahren nach einem oder mehreren der Ansprüche 1 bis 9, wobei die sprühgetrocknete Feststoffdispersion 1 bis 99 Gew.-% des Dispersionspolymers umfasst, wobei die Gew.-% auf das Gewicht der sprühgetrockneten Feststoffdispersion bezogen sind.

**11.** Verfahren nach einem oder mehreren der Ansprüche 1 bis 10, wobei der kombinierte Gehalt an Wirkstoff und Dispersionspolymer in der sprühgetrockneten Feststoffdispersion 65 bis 100 Gew.-% beträgt, wobei die Gew.-% auf das Gewicht der sprühgetrockneten Feststoffdispersion bezogen sind.

**12.** Verfahren nach einem oder mehreren der Ansprüche 1 bis 11, wobei der Wirkstoff eine biologisch aktive Verbindung ist.

**13.** Verfahren nach einem oder mehreren der Ansprüche 1 bis 12, wobei der Wirkstoff ein Arzneistoff, ein Medikament, ein Pharmazeutikum, ein Therapeutikum, ein Nutrazeutikum, ein pharmazeutischer Wirkstoff ist.

**14.** Verfahren nach einem oder mehreren der Ansprüche 1 bis 13, wobei der Wirkstoff einen basischen pKa von 5 oder höher aufweist.

**15.** Verfahren nach einem oder mehreren der Ansprüche 1 bis 14, wobei das Dispersionspolymer ein oder mehrere Dispersionspolymere umfasst.

**16.** Verfahren nach einem oder mehreren der Ansprüche 1 bis 15, wobei das Dispersionspolymer ein pharmazeutisch verträgliches Dispersionspolymer ist.

**17.** Verfahren nach einem oder mehreren der Ansprüche 1 bis 16, wobei das Dispersionspolymer Hydroxypropylmethylcelluloseacetatsuccinat, Hydroxypropylmethylcellulosephthalat, Hydroxypropylmethylcellulose, Hydroxypropylcellulose, Celluloseacetatphthalat, Carboxymethylethylcellulose, Polyvinylpyrrolidon, Poly(vinylpyrrolidon-co-vinylacetat), Poly(methacrylsäure-co-methylmethacrylat), Poly(methacrylsäure-co-ethylacrylat) oder eine beliebige Kombination davon ist.

**18.** Verfahren nach einem oder mehreren der Ansprüche 1 bis 17, wobei das Dispersionspolymer HPMCAS oder PMMAMAA ist.

**19.** Verfahren nach einem oder mehreren der Ansprüche 1 bis 18, wobei die SDD restliche Essigsäure umfasst; der Gehalt an restlicher Essigsäure in der sprühgetrockneten Feststoffdispersion 5.000 ppm oder weniger beträgt, wobei die ppm auf das Gewicht der sprühgetrockneten Feststoffdispersion bezogen sind.

**20.** Verfahren nach einem oder mehreren der Ansprüche 1 bis 19, wobei die SDD Restlösungsmittel umfasst, der Gehalt an Restlösungsmittel in der sprühgetrockneten Feststoffdispersion 5.000 ppm oder weniger beträgt, wobei die ppm auf das Gewicht der sprühgetrockneten Feststoffdispersion bezogen sind.

**21.** Verfahren nach einem oder mehreren der Ansprüche 1 bis 6 und 9 bis 20, wobei das Lösungsmittel aus $C_{1-3}$-Alkanol besteht.

**22.** Verfahren nach einem oder mehreren der Ansprüche 1 bis 21, wobei das Lösungsmittel aus $C_{1-3}$-Alkanol und Wasser besteht.

**23.** Sprühgetrocknete Feststoffdispersion, erhältlich durch das Verfahren nach einem der Ansprüche 1 bis 22.

**Revendications**

1. Procédé de préparation d'une dispersion solide séchée par pulvérisation d'un agent actif, qui est une base de Bronstedt organique, comprenant :

    a. la combinaison d'un agent actif, d'un polymère de dispersion, d'acide acétique et d'un solvant pour former une solution de pulvérisation, dans lequel

        i. le solvant comprend un alcanol en $C_{1-3}$, la quantité d'alcanol en $C_{1-3}$ dans le solvant est d'au moins 50 % en poids, le % en poids étant basé sur le poids du solvant ;
        ii. l'agent actif est sous sa forme de base libre lorsqu'il est combiné avec l'acide acétique et le solvant pour former la solution de pulvérisation et, sous sa forme de base libre, a un pKa basique de 4 ou plus, et l'agent actif a une solubilité de 40 mg/ml ou moins dans le solvant,
        iii. la solution de pulvérisation n'est pas une solution sursaturée de l'agent actif dans le solvant et l'acide acétique ;

    b. le séchage par pulvérisation de la solution de pulvérisation pour former une dispersion solide séchée par pulvérisation comprenant l'agent actif et le polymère de dispersion ;

    dans lequel la solution de pulvérisation ne comporte qu'une seule phase liquide.

2. Procédé selon la revendication 1, dans lequel la quantité d'agent actif par rapport au solvant est supérieure à la solubilité dudit agent actif dans le solvant en l'absence d'acide acétique.

3. Procédé selon la revendication 1 ou 2, dans lequel la quantité d'agent actif dans la solution de pulvérisation est d'au moins 0,5 % en poids, le % en poids étant basé sur le poids de la solution de pulvérisation.

4. Procédé selon une ou plusieurs des revendications 1 à 3, dans lequel la quantité d'acide acétique est de 1 à 50 équivalents sur la base de la quantité molaire d'agent actif.

5. Procédé selon une ou plusieurs des revendications 1 à 3, dans lequel la quantité d'acide acétique est de 0,05 à 50 % en poids, le % en poids étant basé sur le poids du solvant.

6. Procédé selon une ou plusieurs des revendications 1 à 5, dans lequel le solvant est le méthanol, l'éthanol ou l'isopropanol.

7. Procédé selon une ou plusieurs des revendications 1 à 6, dans lequel le solvant comprend en outre de l'eau.

8. Procédé selon la revendication 7, dans lequel le rapport pondéral alcanol en $C_{1-3}$ : eau dans le solvant peut être de 99 : 1 à 60 : 40.

9. Procédé selon une ou plusieurs des revendications 1 à 8, dans lequel la dispersion solide séchée par pulvérisation comprend de 1 à 99 % en poids d'agent actif, le % en poids étant basé sur le poids de la dispersion solide séchée par pulvérisation.

10. Procédé selon une ou plusieurs des revendications 1 à 9, dans lequel la dispersion solide séchée par pulvérisation comprend de 1 à 99 % en poids du polymère de dispersion, le % en poids étant basé sur le poids de la dispersion solide séchée par pulvérisation.

11. Procédé selon une ou plusieurs des revendications 1 à 10, dans lequel la teneur combinée en agent actif et en polymère de dispersion de la dispersion solide séchée par pulvérisation est de 65 à 100 % en poids, le % en poids étant basé sur le poids de la dispersion solide séchée par pulvérisation.

12. Procédé selon une ou plusieurs des revendications 1 à 11, dans lequel l'agent actif est un composé biologiquement actif.

13. Procédé selon une ou plusieurs des revendications 1 à 12, dans lequel
l'agent actif est un médicament, un remède, un agent pharmaceutique, un agent thérapeutique, un nutraceutique, un ingrédient pharmaceutique actif.

14. Procédé selon une ou plusieurs des revendications 1 à 13, dans lequel
l'agent actif a un pKa basique de 5 ou plus.

15. Procédé selon une ou plusieurs des revendications 1 à 14, dans lequel
le polymère de dispersion comprend un ou plusieurs polymères de dispersion.

16. Procédé selon une ou plusieurs des revendications 1 à 15, dans lequel

le polymère de dispersion est un polymère de dispersion pharmaceutiquement acceptable.

17. Procédé selon une ou plusieurs des revendications 1 à 16, dans lequel
le polymère de dispersion est l'acétate succinate d'hydroxypropylméthylcellulose, le phtalate d'hydroxypropylméthylcellulose, l'hydroxypropylméthylcellulose, l'hydroxypropylcellulose, l'acétate phtalate de cellulose, la carboxyméthyléthylcellulose, la polyvinylpyrrolidone, le poly(vinylpyrrolidone-co-acétate de vinyle), le poly(acide méthacrylique-co-méthacrylate de méthyle), le poly(acide méthacrylique-co-acrylate d'éthyle), ou toute combinaison de ceux-ci.

18. Procédé selon une ou plusieurs des revendications 1 à 17, dans lequel
le polymère de dispersion est HPMCAS ou PMMAMAA.

19. Procédé selon une ou plusieurs des revendications 1 à 18, dans lequel
le SDD comprend de l'acide acétique résiduel ; la teneur en acide acétique résiduel de la dispersion solide séchée par pulvérisation est de 5 000 ppm ou moins, le ppm étant basé sur le poids de la dispersion solide séchée par pulvérisation.

20. Procédé selon une ou plusieurs des revendications 1 à 19, dans lequel
le SDD comprend un solvant résiduel, la teneur en solvant résiduel de la dispersion solide séchée par pulvérisation est de 5 000 ppm ou moins, le ppm étant basé sur le poids de la dispersion solide séchée par pulvérisation.

21. Procédé selon une ou plusieurs des revendications 1 à 6 et 9 à 20, dans lequel
le solvant est constitué d'alcanol en $C_{1-3}$.

22. Procédé selon une ou plusieurs des revendications 1 à 21, dans lequel
le solvant est constitué d'alcanol en $C_{1-3}$ et d'eau.

23. Dispersion solide séchée par pulvérisation pouvant être obtenue par le procédé selon l'une quelconque des revendications 1 à 22.

Fig 1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019220282 A1
- WO 2007060384 A2
- CN 110037990 A
- WO 2015138837 A1
- US 20070218012 A1
- WO 2013105894 A1

**Non-patent literature cited in the description**

- *CHEMICAL ABSTRACTS,* 302962-49-8
- *CHEMICAL ABSTRACTS,* 184475-35-2
- *CHEMICAL ABSTRACTS,* 71138-97-1